# EUROPEAN PATENT SPECIFICATION

(11) **EP 2 068 863 B1**
(45) Date of publication and mention of the grant of the patent: **15.12.2010**
(21) Application number: 07786654.9
(22) Date of filing: 10.08.2007
(51) Int. Cl.: A61K 31/343, A61P 25/22, A61P 25/24

(54) **LIGUSTILIDE FOR THE TREATMENT OF DISORDERS OF THE CENTRAL NERVOUS SYSTEM**
LIGUSTILID ZUR BEHANDLUNG VON ERKRANKUNGEN DES ZENTRALEN NERVENSYSTEMS
LIGUSTILIDE POUR LE TRAITEMENT DES MALADIES DU SYSTEME NERVEUX CENTRAL

(30) Priority: 11.08.2006 EP 06016770
(43) Date of publication of application: 17.06.2009
(73) Proprietor: DSM IP Assets B.V., 6411 TE Heerlen (NL)
(72) Inventor: DE SAIZIEU, Antoine, 68350 Brunstatt (FR); GORALCZYK, Regina, 79639 Grenzach-Wyhlen (DE); SCHUELER, Goede, 79591 Eimeldingen (DE)
(74) Representative: Schwander, Kuno
(86) International application number: PCT/EP2007/007086
(87) International publication number: WO 2008/017491

(56) References cited:
- WO-A-02/40024
- WO-A-95/00157
- WO-A-2004/100945
- US-A- 5 788 986
- DATABASE WPI Week 199329 Derwent Publications Ltd., London, GB; AN 1993-232265 XP002403960 & JP 05 155768 A (KAO CORP) 22 June 1993 (1993-06-22)
- JOURNAL OF FOOD SCIENCE, vol. 43, no. 1, 1978, pages 143-144, XP000560022 ISSN: 0022-1147
- DATABASE WPI Week 200510 Derwent Publications Ltd., London, GB; AN 2005-091657 XP002403961 & WO 2005/002568 A1 (null) 13 January 2005 (2005-01-13)
- DATABASE BIOSIS [Online] BIOSCIENCES INFORMATION SERVICE, PHILADELPHIA, PA, US; 1997, HU DUN ZHANG LI-YING ET AL: "Effect of dl-3-n-butylphthalide on memory disturbance induced by focal cerebral ischemia in rats" XP002403951 Database accession no. PREV199799477394 & ZHONGGUO YAOLIXUE YU DULIXUE ZAZHI, vol. 11, no. 1, 1997, pages 14-15, ISSN: 1000-3002
- DATABASE EMBASE [Online] ELSEVIER SCIENCE PUBLISHERS, AMSTERDAM, NL; 1997, HU D ET AL: "Effect of dl-3-n-butylphthalide on memory disturbance induced by focal cerebral ischemia in rats" XP002403952 Database accession no. EMB-1997092441 & CHINESE JOURNAL OF PHARMACOLOGY AND TOXICOLOGY 1997 CHINA, vol. 11, no. 1, 1997, pages 14-16, ISSN: 1000-3002
- DATABASE CA [Online] CHEMICAL ABSTRACTS SERVICE, COLUMBUS, OHIO, US; 1994, NAITO, TAKASHI ET AL: "Isolation of phthalide derivatives from Senkyu and pharmaceutical compositions for improvement of brain function" XP002403953 retrieved from STN Database accession no. 1994:173452 & JP 05 247022 A2 (TSUMURA & CO, JAPAN) 24 September 1993 (1993-09-24)
- DATABASE WPI Week 200577 Derwent Publications Ltd., London, GB; AN 2005-749412 XP002402177 & CN 1 569 848 A (INST CHINESE MATERIA MEDICA CHINA ACAD T) 26 January 2005 (2005-01-26)
- DATABASE WPI Week 198939 Derwent Publications Ltd., London, GB; AN 1989-282536 XP002402179 & JP 01 207233 A (KAKURITSU EISEI SHIKENJO) 21 August 1989 (1989-08-21)
- DATABASE WPI Week 198914 Derwent Publications Ltd., London, GB; AN 1989-103886 XP002457615 & JP 01 050818 A (TSUMURA JUNTENDO KK) 27 February 1989 (1989-02-27)

## Description

The present invention refers to ligustilde use in the method of treatment of certain disorders connected to impaired neurotransmission, as well as to dietary and pharmaceutical compositions containing such compounds, and to their uses.

It is well known that impaired neurotransmission, e.g. low neurotransmitter levels, is connected to mental diseases such as depression and generalized anxiety disorders (GAD), and increased susceptibility to stress.

JP 05 155768 (Kao Corp.) and Bjeldanes et al. 1978 J. Food Sci. 43(1):143 both reach that 3-n-butylphthalid can induce sleep.

Naito Takashi et al JP 05-247022 (Tsumura & Co) reach that various compounds including ligustilide can improve brain function in Alzheimer's Disease patients.

Compounds that increase neurotransmitter levels in the brain and thus enhance their transmission, exhibit therefore antidepressant properties as well as beneficial effects on a variety of other mental disorders ("Neurotransmitters, drugs and brain function" R.A. Webster (ed.), John Wiley & Sons, New York, 2001, p. 187-211, 289-452, 477-498). The main neurotransmitters are serotonin, dopamine, noradrenaline (= norepinephrine), acetylcholine, glutamate, gamma-amino-butyric acid, as well as neuropeptides. Increase in neurotransmission is achieved by increasing the concentration of the neurotransmitter in the synaptic cleft thus making it available for increased or prolonged neurotransmission through inhibition of re-uptake into the pre-synaptic nerve end, or by preventing neurotransmitter catabolism by inhibition of degrading enzymes such as monoaminooxidase A and B.

Tricyclic antidepressant compounds (TCAs) such as imipramine, amitriptyline, and clomipramine e.g. inhibit the re-uptake of serotonin and noradrenaline. They are widely retarded as among the most effective antidepressants available, but they have a number of disadvantages because they interact with a number of brain receptors, e.g. with cholinergic receptors. Most importantly, TCAs are not safe when taken in overdose, frequently showing acute cardiotoxicity.

Another class of antidepressant drugs are the so-called SSRIs (selective serotonin re-uptake inhibitors) including fluoxetine, paroxetine, sertraline, citalopram, fluvoxamine that block the serotonin transporter (SERT), a high affinity sodium chloride-dependent neuro-transmitter transporter that terminates serotonergic neurotransmission by uptake of serotonin. They have been proven as effective in the treatment of depression and anxiety, and are usually better tolerated than TCAs. These medications are typically started at low dosages and may be increased until they reach a therapeutic level. A common side effect is nausea. Other possible side effects include decreased appetite, dry mouth, sweating, infection, constipation, yawn, tremor, sleepiness and sexual dysfunction.

In addition, compounds that prevent the catabolism of neurotransmitters more broadly by inhibiting the monoaminooxidases (MAOs) A and B exhibit antidepressant effects. MAOs catalyse the oxidation of amino group containing neurotransmitters such as serotonin, noradrenaline, and dopamine.

Furthermore, modulators of neurotransmission exert pleiotropic effects on mental and cognitive functions. These functions differ clearly from those effects reported for food/plant constituents with antioxidant function which exert a neuroprotective effect via reduction of oxidative stress.

In addition, patients often suffer either as a comorbidity to depression, or by itself from generalized anxiety syndrome (GAD). GAD as a highly prevalent anxiety condition and chronic illness in primary care (∼10% of patients) (Wittchen, et al 2005. European Neuro-psychopharm. 15:357 - 376). Patients present themselves to their primary care physician with multiple physical symptoms. GAD is characterized by chronic tension, and anxious worrying and tension (>6 months), which are disabling and uncontrollable, and accompanied by a characteristic hypervigilance syndrome (including restlessness, muscle tension, and sleep problems). If untreated, GAD runs a chronic, fluctuating course and tends to get more severe with age. GAD patients suffer from subsyndromal depression and contribute to the highest overall direct and indirect health economic burden of all anxiety and depressive disorder. Despite high GAD incidence, few sufferers are diagnosed, prescribed medication, or receive psychiatric referral-simple diagnostic tools to aid patient recognition and monitoring are needed. Regardless of specific diagnosis, physicians require effective GAD-sumptom treatments. SSR is such as paroxetine are effective for GAD treatment [Stocchi et al. 2003 J Clin Psych, 63(3):250-258]. Also, systematic reviews and placebo-controlled RCTs (Randomized Clinical Trials) indicate that some SSRIs (escitalopram, paroxetine and sertraline), the SNRI (Selective Norepinephrine Reuptake Inhibitors) venlafaxine, some benzodiazepines (alprazolam and diazepam), the tricyclic imipramine, and the 5-HTI A partial agonist buspirone are all efficacious in acute treatment. In general, the effect of treatment is often moderate and symptoms reappear when the treatment period is discontinued. Therefore, a continuous long-term treatment or prevention with compounds which have less side effects as SSRIs and can be taken over long time periods might by favourable over drug treatment.

There is a need for compounds for the treatment or prevention of mental diseases and/or disorders which do not show the negative side effects of known antidepressants. Many patients are interested in alternative therapies which could minimize the side effects associated with high-dose of drugs and yield additive clinical benefits. Severe depression is a long feasting and recurring disease, which is usually poorly diagnosed. Furthermore many patients suffer from mild or middle severe depression. Thus, there is an increasing interest in the development of compounds as well as pharmaceutical and/or dietary compositions that may be used to treat mental diseases/disorders or to prevent the development of mental diseases/disorders such as depression and disthymia in people at risk, and to stabilize mood.

It is know that imbalanced mood is connected to impaired, i.e., reduced neurotransmission, and that this also leads to increased susceptibility to stress.

Thus, mood is influenced by neurotransmitter biosynthesis, neurotransmitter processing, neurotransmitter storage, neurotransmitter release, neurotransmitter re-uptake and neurotransmitter receptor binding, especially wherein serotonin is the neurotransmitter. Imbalanced mood may manifest itself in animals including humans as tension, sadness, unhappiness/discontent, irritability and dysphoria, and/or as a disturbance of behaviour, emotions and thinking processes.

Thus, there is also a need for compounds to harmonize or normalize mood and achieve emotional balance to cope with daily life stress and to maintain physical and psychological performance.

Mood disorders and occupational stress also lead to consecutive sleep disorders, (insomnia) delayed sleep onset and low sleep quality, disturbances in circadian rhythms (so-called bio-rhythm). These conditions are often chronic and can persist over long time. Also, deregulation of circadian rhythms induced by long-distance flights (jet-lag) as well as by shirt-working can cause similar symptoms and distress. Therefore, treatment with dietary supplementation to alleviate and prevent symptoms associated with the sleep disorders, as well as to maintain the normal circadian rhythm (an animal or human is used to), and/or to alleviate and prevent symptoms associated with a disturbed circadian rhythm, such as impairment of cognitive function and memory, mental and physical fatigue, dreaminess, is warranted to improve the overall quality of life and benefiting vital energy of a person in need thereof.

### DESCRIPTION OF THE INVENTION

Surprisingly, it has been found ligustilide is an effective serotonin and noradrenaline re-uptake inhibitor, and inhibitor of monaminooxidase A and B. Thus it has similar properties to currently commercialized antidepressants drugs like SSRIs (selective serotonin-reuptake inhibitiors), SNRIs (selective noradrenatine-reuptaice inhibitiors) and tricyclic amines)

The present invention refers especially to the non-therapeutic use of ligustilide as mood balancing agents and stress relievers, for mental well-being as well as to helping to reduce the risk of mood swings, for helping to retain a positive mood and for supporting cognitive wellness, for normalizing the sleep pattern (also called sleep architecture), shorten the time to sleep onset, in general for helping to promote and maintain a good sleep quality, as well as (dietary) compositions and fortified food/feed/beverages containing them, and their uses.

Additionally the compound of this invention has veternary uses, including: preventing stress in farm animals in mass production lifestock husbandry and/or during transport to slaughter; preventing quality loss of meat of the farm animals during transport to slaughter; for preventing feather picking and cannibalism amongst poultry; and for reduction of stress in pets.

According to the present invention this demand is met with ligustilide.

Thus, in one aspect the invention relates to the use of ligustilide, as a mood balancing agent and/or stress reliever in animals including humans.

The animals are especially humans, pet or companion animals (preferably cats and dogs), farm animals (preferably poultry, cattle, sheep, goat and swine) and animals for the fur industry such as minks, foxes and hares, as well as animals used for aquaculture such as fish like salmon and trout as well as crustaceous like shrimp.

In one preferred embodiment of the present invention ligustilide is used for preventing stress in pet and farm animals, in mass production lifestock husbandry, during transport to slaughter and/or for preventing losses in milk or egg production, and quality loss of meat of said farm animals during transport to slaughter.

In another preferred embodiment of the present invention the ligustilide is used for preventing feather picking and cannibalism amongst poultry and/or for preventing losses of meat quality and egg production accompanied by it and in general.

In a further preferred embodiment of the present invention the ligustilide is used for maintaining the circadian rhythm in humans, for alleviating and/or for preventing the symptoms associated with a disturbed circadian rhythm in humans. Thus, mood is stablilized and an emotional balance is achieved to cope with daily life stress and to maintain physical and psychological performance. Furthermore, the symptoms associated with a disturbed circadian rhythm such as impairment on cognitive function and memory, and mental and physical fatigue are alleviated and/or prevented so that the overall quality of life is improved and the persons to whom such compounds are administered benefit from maintaining vital energy. Also, deregulation of circadian rhythms induced by long-term flights (jet-lag) as well as by shift-working and the symptoms associated with it are alleviated and/or prevented.

Thus, imbalanced mood which may manifest as tension, sadness, unhappiness/discontent and irritability, dysphoria, and/or as a disturbance of behaviour, emotions and thinking processes, is prevented by administering ligustilide to animals including humans.

In another aspect, the invention relates to the use of a ligustilide for the treatment of a disorder connected to impaired or reduced neuro-transmission or for the manufacture of a composition for use in the method of treatment of a disorder connected to impaired neurotransmission, particularly for the manufacture of an antidepressant, a reducer of obsessive-compulsive behaviour, a relaxant, a sleep improver and/or a insomnia alleviator, as well as for non-therapeutic use as mood balancing agent, as mood/vitality improver, as stress reliever, as condition improver and/or as reducer of tension, sadness, unhappiness/discontent, irritability and dysphoria.

In still another aspect, the invention relates to compositions, particularly to dietary compositions containing ligustilide as well as to pharmaceutical compositions containing ligustilide and a conventional pharmaceutical carrier for use in the treatment of disorders connected to impaired neurotransmission.

Animals in the context of the present invention include humans and encompass mammals, fish and birds. Preferred "animals" are humans, pet or companion animals, farm animals, animals for the fur industry and in aquaculture. More preferred "animals" are humans, pets and farm animals.

Examples for pet animals are dogs, cats, birds, aquarium fish, guinea pigs, (jack) rabbits, hares and ferrets.

Examples of farm animals are fish (such as e.g. salmon and trout), aqua culture animals (such as e.g. shuimp), pigs, horses, ruminants (cattle, sheep and goat) and poultry (such as e.g. geese, chicken, broiler, laying hens, quails, ducks, turkeys).

Examples of animals for the fur industry are minks, foxes and hares.

In a preferred embodiment of the present invention ligustilide is used for preventing stress in farm animals, in mass production lifestock husbandry, during transport to slaughter and/or for preventing quality loss of meat of said farm animals during transport to slaughter. Said farm animals are especially poultry (such as e.g. geese, broiler, laying hens, quails, ducks, chicken, turkeys), cattle, sheep, goat and swine.

In another preferred embodiment of the present invention ligustilide is used in pet animals for reduction of stress, tension and aggressiveness and compulsive behavior under stressfully conditions such as separation, change or loss of the owner, during holiday separation and husbandry in so called "animal hotels", husbandry in animal shelter stations, and other conditions of dense husbandry and breeding.

In another preferred embodiment of the present invention ligustilide is used poultry (such as e.g. geese, broiler, laying hens, quails, ducks, chicken, turkeys) for preventing feather picking and cannibalism resuiting e.g. in losses of meat quality and egg production.

In a further preferred embodiment of the present invention ligustilide is used in humans for maintaining the circadian rhythm in humans, for alleviating and/or preventing the symptoms associated with a disturbed circadian rhythm in humans.

The term "ligustilide" also encompasses any material or extract of a plant containing ligustilide preferably in an amount of at least 50 weight-%, more preferably in an amount of from 70 to 90 weight-%, most preferably in an amount of at least 90 weight-%, based on the total weight of the plant material or extract. The terms "material of a plant" and "plant material" used in the context of the present invention mean any part of a plant.

Ligustilide may be isolated by methods known in the art [see, e.g. Beck J.et al 1995 Natural Products 58(7): 147-1055] from various plants such as *Angelica glauca, Angelica acutiloba, Angelica sinensis, Angelicae dahuricae, Apium graveolens, Ligusticum acutilobum, Ligusticum officinale, Ligusticum sinense, Liguslicum wallichii, Ligusticum chuanxiong, Cnidium officinale, Rhizoma chuanxiong, Pleurospermum hookeri, Trachyspermum roxburghianum, Meum athamancicum, Lomatium torreyi, Scutellaria baicalensis, Opopanax chironium, Cenolophium denudatum, Coriandrum sativuum,* and *Silaum silaus.* Therefore, any material, extract or oleoresin of these plants or any other plant material or extract containing lingustilide, especially in an amount of at least 50 weight-%, preferably in an amount of from 70 to 90 weight-%, more preferably in an amount of at least 90 weight-%, based on the total weight of the plant material or extract, is also encompassed by the expression "ligustilide." The compounds used herein may also be of synthetic origin.

Supercritical fluid extract/distillation is a preferred method for extracting Ligusticum.

Beside the pure ligustilide especially preferred are plant materials and plant extracts containing at least 50 wight-%, more preferably from 70 to 90 weight-%, most preferably at least 90 weight-%, of these compouds, based on the total weight of the plant material/extract. Extracts of whole plants or parts thereof, e.g., of leaves, roots or seed are commercially available or can be obtained in accordance with methods well-known in the art using solvents like methanol, ethanol, ethyl acetate, dimethyl ether, n-hexane, methylenechloride or, preferably, with supercritical fluids like carbon dioxide (pure or in mixture with other solvents such as alcohols) or dinitrogen oxide. From the extracts single compounds or more or less pure mixtures can be obtained by distillation and fractionation, preferably under reduced pressure. Alternatively, ligustilide can be synthesized by methods well-known in the art and mixed together in any desired quantities.

In a preferred embodiment of the present invention the ligustilide is administered in form of a ligusticum distillate, containing:
Z-ligustilide,
   preferably the amount of Z-ligustilide is:
   in an amount in the range of from at least 30 to 60 weight-%;
   more preferably at least 40 weight-%,
   even more preferably at least 50 weight-%.

Amounts based on the total weight of the distillate/mixture.

The dietary compositions according to the present invention may further contain protective hydrocolloids, binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

The term "dietary compositions" comprises any type of nutrition such as (fortified) food/feed and beverages including also clinical nutrition, and also dietary supplements.

Beside a pharmaceutically acceptable carrier and at least one compound of the general formula I with the definitions of X, R¹ - R⁵ and the preferred ones as given above, the pharmaceutical compositions according to the present invention may further contain conventional pharmaceutical additives and adjutants, excipients or diluents, including, but not limited to, water, gelatin of any origin, vegetable gums, ligninsulfonate, talc, sugars, starch, gum arable, vegetable oils, polyalkylene glycols, favoring agents, preservatives, stabilizers emulsifying agents, buffers, lubricants, colorants, wetting agents, fillers, and the like. The carrier material can be organic or inorganic inert carrier material suitable for oral/parenteral/injectable administration.

The dietary and pharmaceutical compositions according to the present invention may be in any galenic form that is suitable for administration to the animal body including the human body, especially in any form that is conventional for oral administration, e.g. in solid form such as (additives/supplements for) food or feed, food or feed premix, fortified food or feed, tablets, pills, granules, dragées, capsules, and effervescent formulations such as powders and tablets, or in liquid form such as solutions, emulsions or suspensions as e.g. beverages, pastes and oily suspensions. The pastes may be filled into hard or soft shell capsules. Examples for other application forms are forms for transdermal, parenteral or injectable administration. The dietary and pharmaceutical compositions may be in the form of controlled (delayed) release formulations.

Examples of food are dairy products such as yoghurts.

Examples for fortified food are cereal bars, bakery items such as cakes and cookies.

Beverages encompass non-alcoholic and alcoholic drinks as well as liquid preparations to be added to drinking water and liquid food. Non-alcoholic drinks are e.g. soft drinks, sport drinks, fruit juices, lemonade, near-water drinks (i.e. water based drinks with a low calorie content), teas and milk based drinks. Liquid food are e.g. soups and dairy products (e.g. muesli drinks).

In the context of this invention "treatment" also encompasses co-treatment as well as prevention. "Prevention" can be the prevention of the first occurrence (primary prevention) or the prevention of a reoccurence (secondary prevention).

In the context of this invention the term "disorder" also encompasses diseases.

Pharmaceutical and dietary compositions for the treatment of disorders connected to impaired neurotransmission encompass their use as antidepressants, mood/vitality improvers, stress relievers, condition improvers, anxiety reducers and obsessive-compulsive behaviour reducers, relaxants, sleep improvers and/or insomnia alleviators, normalizer of circadian rhythm (biorhythm). They all may improve, enhance and support the physiological neurotransmission, especially in the central nervous system, and therefore may alleviate mental malfunction.

Antidepressants are medicaments for treating mental, behavioural and emotional/affective, neurotic, neurodegenerative, eating and stress related disorders such as e.g. unipolar depression, bipolar depression, acute depression, chronic depression, subchronic depression, dysthymia, postpartum depression, premenstrual dysphoria/syndrom (PMS), climacteric depressive symptoms, aggression, attention deficit disorders (ADS), social anxiety disorders, seasonal affective disorders, anxiety (disorders) such as generalized anxiety disorder (GAD), fibromyalgia syndrome, chronic fatigue, sleep disorders (insomnia), post-traumatic stress disorders, panic disorders, obsessive compulsive disorders, restless leg syndrome, nervousness, migraine/primary headaches and pain in general, emesis, bulimia, anorexia nervosa, binge eating disorder, gastrointestinal disorders, burn out syndrome, irritability and tiredness.

Antidepressants can also be used for (the manufacture of companions for) primary and secondary prevention and/or the treatment of neurocognitive impairment. Furthermore they are also effective in the treatment of depressive symptoms or other symptoms related to disturbed neurotransmission occurring as comorbidity in chronic diseases such as cardiovascular diseases, strokes, cancer, Alzheimer disease, Parkinson disease, and others.

Ligustilide as well as (mixtures of) plant materials and plant extracts (essentially) containing them (especially in an amount of at least 30 weight-%, preferably in an amount of at least 50 weight-%, more preferably in an amount of from 70 to 90 weight-%, most preferably in an amount of at least 90 weight-%, based on the total weight of the plant material or extract) and dietary/pharmaceutical compositions containing them are thus suitable for the treatment of animals including humans.

Especially companion (pet) animals and livestock (farm) animals can be in conditions in need of enhanced or improved neurotransmission. Such conditions e.g. occur after capture or transport or with housing, when the animals develop analogous disorders and are distressed or aggressive, or display stereotypic behaviour, or anxiety and obsessive-compulsive behaviour.

"Mood improver" or "emotional wellness booster" or "vitality improver" means that the mood of a person treated with it is enhanced, that the self esteem is increased and/or that negative thoughts and/or negative tension are/is reduced. It also means the emotions are balanced and/or that the general, especially the mental, well being and vitality is improved or maintained, as well as that the risk of mood swings is (helped to be) reduced and that the positive mood is (helped to be) retained.

Ligustilide can also be used in general as anxiety reducer and/or obsessive-compulsive behaviour reducer for animals including humans, preferably for humans, pet animals and farm animals.

Anxiety reducer means that chronic tension and anxious worrying and tension are alleviated and relieved. Hypervigilance syndrome, including restlessness, muscle tension, and sleep problems are relieved. Social and other phobias are resolved. In general, the social environment is experienced less threatening. The person is emotionally relaxed, experiences comport and enjoys company and contact to other people.

"Relaxant" or "sleep improver" or "insomnia alleviator" means improving sleep onset (shortening time to enter sleep, lag time to sleep) and helping a person to easily enter sleep, normalize sleep patterns, or to maintain an undisrupted sleep over the night. It also means to correct circadian rhythm associated sleep disturbances due to jet-lag or shaft work, and to prevent and abolish the symptoms associated with sleeplessness, i.e. impairment of cognitive function and memory, mental and physical fatigue, dreaminess, and improve overall quality of life and vital energy.

Ligustilide as well as compositions comprising an effective dose of are useful for the treatment, prevention and the alleviation of stress related symptoms, for the treatment, prevention and alleviation of symptoms related to working overload, exhaustion and/or burn out, for the increase of the resistance or tolerance to stress and/or to favor and facilitate the relaxation in normal healthy individuals i.e. such compositions have an effect as "stress reliever".

A further embodiment of the present invention relates to the use of ligustilide and to the use of compositions comtaining it (plant materials/extracts; dietary/pharmaceutical compositions) as a "condition improver", i.e. as means to reduce irritability and tiredness, to reduce or prevent or alleviate physical and mental fatigue, and to increase energy in more general terms, especially to increase the brain energy production, in diseased or normal healthy individuals. Moreover for cognition improvement in general, and especially for maintenance or improvement of attention and concentration, of the memory and of the capacity for remembering, of the learning ability, of the language processing, of problem solving and of intellectual functioning; for improvement of the short-term memory; for increasing the mental alertness any sharpness, capability to focus; for enhancing the mental vigilance; for reducing the mental fatigue; for supporting cognitive wellness, for maintaining balanced cognitive function, for the regulation of hunger and satiety as well as for the regulation of motor activity.

For humans a suitable daily dosage of ligusticum for the purposes of the present invention may be within the range from 0.001 mg per kg body weight to about 20 mg per kg body weight per day. More preferred is a daily dosage of about 0.01 to about 10 mg per kg body weight, and especially preferred is a daily dosage of about 0.05 to 5.0 mg per kg body weight. The amount of a plant material or plant extract containing ligusticum can be calculated accordingly.

In solid dosage unit preparations for humans, the ligusticum is suitably present in an amount from about 0.1 mg to about 1000 mg, preferably from about 1 mg to about 500 mg per dosage unit.

In dietary compositions, especially in food and beverages for humans, the ligusticum, is suitably present in an amount of from about 0.0001 (1 mg/kg) to about 5 weight-% (50 g/kg), preferably from about 0.001 % (10 mg/kg)to about 1 weight-%, (10 g/kg) more preferably from about 0.01 (100 mg/kg) to about 0.5 weight-% (5 g/kg), based upon the total weight of the food or beverage.

In food and drinks in a preferred embodiment of the invention the amount of ligusticum is 10 to 30 mg per serving, i.e. 120 mg per kg food or drink.

For animals excluding humans a suitable daily dosage of ligusticum for the purposes of the present invention may be within the range from 0.001 mg per kg body weight to about 1000 mg per kg body weight per day. More preferred is a daily dosage of about 0.1 mg to about 500 mg per kg body weight, and especially preferred is a daily dosage of about 1 mg to 100 mg per kg body weight.

For companion (pet) animals the same daily dosages as for humans are suitable.

The invention is illustrated further by the following examples.

### Examples

The 3-n-butylphthalide used in the experiments described below was obtained from Advanced Synthesis, PO Box 437920, San Ysidro, CA 92173. The senkyunolides and cnidilide were obtained from AnalytiCon Discovery GmbH, Hermannswerder Haus 17, 14473 Potsdam, Germany, and the ligustilide was synthesized by chemists of DSM Nutritional Products Ltd., Kaiseraugst, Switzerland. All compounds were > 95% pure.

### Example 1:

### Serotonin uptake inhibition and labeled citalopram displacement by compounds of the formula I.

HEK-293 cells stably expressing the human serotonin re-uptake transporter (hSERT) were obtained from R. Blakely, Vanderbilt University, USA. The cells were routinely grown in Dulbeco's Modified Eagles Medium, purchased from Bioconcept, Allschwil, Switzerland containing 10% fetal calf serum, penicillin, streptomycin, L-glutamine and the antibiotic G418 and passaged by trypsinisation. One day prior to the assay cells were seeded in the above mentioned medium. Immediately prior to the assay the medium was replaced by Krebs Ringers bicarbonate buffer, purchased from Sigma Chemicals Ltd., supplemented with 35 µM pargyline, 2.2 mM CaCl₂, 1 mM ascorbic acid and 5 mM N-2-hydroxyethylpiperazine-N'-2-ethanesulfonic acid (buffer called "Hepes"). Serotonin uptake into the cells was determined by addition of radio-labeled (3H) serotonin (Amersham Biosciences GE Healthcare, Slough, UK) to a concentration of 20 nM, and incubation for 30 minutes at room temperature. Following removal of unincorporated label by gentle washing three times with the above buffer, incorporated serotonin was quantified by liquid scintillation counting.

The effect of the compounds of formula I on the serotonin uptake was determined by its inclusion in the assay at a range of concentrations between 0.03 and 100 µM for 10 minutes prior to and during the addition of (3H) serotonin / citalopram. Serotonin uptake via the transporter was inhibited by ligustilide, 3-butylphthalide, senkyunolide A, senkyunolide C, senkyunolide B, and cnidilide, respectively, in a dose dependent manner, with IC₅₀ values as shown in Table 2.

**Table 2: Inhibition of serotonin uptake into transacted HEK-293 cells by compounds of formula I.**

| **Substance** | **IC₅₀ for tritiated serotonin uptake.** |
|---|---|
| Ligustilide | 20.4 +/- 5.8 µM (n=4) |
| 3-n-butylphthalide | 35.2 +/- 6.7 µM (n=3) |
| Senkyunolide A | 32.5 +/- 9.1 µM (n=2) |
| Senkyunolide C | 31.4 +/- 10.9 µM (n=2) |
| Senkyunolide B | 75.0 +/- 11.0 µM (n=2) |
| Cnidilide | 40.5 +/- 15.0 µM (n=3) |
| Celery extract | 99.2 +/- 5.8 µg/ml (n=2) |
| Wild celery extract | 29.9 +/- 8.3 µg/ml (n=2) |

Since the experiments were carried out twice or more (n) the average value +/- the standard error in the mean (s.e.m.) is shown.

### Example 2: Monoaminooxidase inhibition by compounds of the formula I

The organic amines p-tyramine or benzylamine were used as substrates for the Monoamine oxidase A (MAO-A) and B (MAO-B) enzymes respectively. The H₂O₂ produced by this reaction was quantified by reaction with vanillic acid, catalysed by horse radish peroxidase (HRP).

The reactions were carried out in polystyrene microtitre plates. The MAO enzymes (final concentration 2 U/ml) were mixed with either p-tyramme (Sigma, final concentration 0.5 mM) or benzylamine (Sigma, final concentration 0.5 mM) as appropriate and the chromogenic solution (containing vanillic acid (Fluka), 4-aminoantipyrine (Fluka) and horse radish peroxidase (Sigma), final concentrations 0.25 mM, 0.125 mM and 1 U/ml respectively) in 0.2 M potassium phosphate buffer pH 7.6. The reactions were followed in a microtitre plate absorbance reader eg Spectramax M5 (Molceular Devices Corporation). Absorbance readings at 495 nm were taken every 15 seconds for 40 minutes and the initial reaction velocties calculated by linear regression using SOFTmaxPro (Molecular Devices Corporation).

The effect of ligustilide, 3-butylphthalide, senkyunolide C and cnidilide on the monoamine oxidase enzymes was determined by their inclusion in the assay at a range of concentrations between 0.03 and 100 µM for 10 minutes prior to and during the incubation with substrate. To determine the effect of the compounds on the HRP catalyzed portion of the reaction, the MAO enzyme was replaced by H₂O₂ (Molecular Probes, finial concentration 0.2 mM). The reactions containing MAO-A and MAO-B were both inhibited by tricyclic diterpenes and their derivatives according to formula 1 in a dose dependent manner, whilst the control reaction, was unaffected. The measured IC50 values for inhibition of monoamine oxidase activity by ligustilide, 3-butylphthalide, senkyunolide C and cnidilide, respectively, are shown in Table 3.

**Table 3: Inhibition of MAO-A and MAO-B by compounds of the formula I**

| **Substance** | **IC50 [µM] for inhibition of MAO-A** | **IC50 [µM] for Inhibition of MAO-B** |
|---|---|---|
| Ligustilide | 15.7 +/- 0.2 s.e.m. n=2 | 24.9 +/- 3.7 s.e.m. n=2 |
| 3-n-butylphthalide | 8.1 +/- 1.2 s.e.m. n=2 | 56. +/- 10.2 s.e.m. n=2 |
| Senkyunolide C | 2.72 +/- 0.16 s.e.m. n=2 | 2.14+/-0.24s.e.m. n=2 |
| Cnidilide | > 100 uM n=2 | 108.8 +/- 17.7 s.e.m. n=2 |

### Example 3 Effect of compounds of the formula I in the Primary Observation (Irwin) Test in the mouse

The method, which detects the first toxic dose, the active dose-range and the principal effects of a test substance on behavior and physiological function, follows that described by Irwin 1968 Psychoparmaco, 13:222-257.

Mice were administered the test substance (3-n-butyiphthalide, ligustilide) and were observed in simultaneous comparison with a control group given vehicle (non-blind conditions). 3 treated groups were compared with the same control at any one time. All animals within a treatment group were observed simultaneously.

Behavioural modifications, physiological and neurotoxicity symptoms, pupil diameter and rectal temperature were recorded according to a standardized observation grid derived from that of Irwin, *supra.* The grid contains the following items: lethality*, convulsions*, tremor*, Straub*, sedation; excitation, abnormal gait* (rolling, tip-toe), jumps*, motor in-coordination*, writes*, piloerection*, stereotypies* (sniffing, chewing, head movements), head twitches*, scratching*, respiration*, aggressiveness*, fear, reactivity to touch, muscle tone, loss of righting reflex, ptosis, exophthalmos, loss of grasping, akinesia, catalepsy, loss of traction, loss of corneal reflux, analgesia, defecation, salivation, lacrimation, pupil diameter (Unit = 1/45 mm) and rectal temperature.

Observations were performed 15, 30, 60, 120 and 180 minutes after administration of the test substance and also 24 hours later. The symptoms marked (*) were observed continuously from 0 to 15 minutes after administration. 5 mice were studied per group.

3-Butylphthalide (98.5% purity) was solubilized in 3 % DMSO, 3 % Tween 80 in saline (0.9 % w/v NaCl) and injected into mice intraperitoneally.

### Results

### 3-n-butylphthalide

At 100mg/kg body weight, 3-n-butylphthalide showed slight sedative effects (in 5/5 mice after 15 and 30 minutes, and 2/5 at 60 minutes) and reduced muscle tone (in 5/5 mice after 15 and 30 minutes, and 3/5 mice after 60 minutes).

At 200 mg/kg body weight it showed moderate sedative effects in 5/5 mice after 60 and 120 minutes), reduced fear and reduced muscle tone after 60 minutes.

At 300 mg/kg body weight, it showed marked sedation in 4/4 mice after 15 minutes and 30 minutes, moderate sedation in 4/4 mice at 60 minutes and 120 minutes, and slight sedation in 4/4 mice at 180 minutes.

3-Butylphthalide at 300mg/kg also reduced fear and muscle tone at 120 minutes.

Overall, 3-butylphthalide showed a dose dependent sedative, fear reducing and muscle relaxant effect.

**Ligustilide** (98.9% purity) at 30 mg/kg induced slight sedation in 3 mice at 30 minutes.

At 100 mg/kg it induced slight sedation in all 5 mice from 15 to 30 minutes and in 3 mice at 60 minutes; it decreased muscle tone in 2 mice at 30 minutes.

At 200 mg/kg sedation was siight-to-moderate in all 5 mice from 15 to 120 minutes and slight in 2 mice at 180 minutes; it decreased muscle tone in all 5 mice at 15 minute, in 4 mice from 30 to 60 minutes, in 2 mice at 120 minutes and in 1 mouse at 180 minutes. At 300 mg/kg sedation was moderate in all 5 mice from 15 to 60 minutes and slight from 120 to 180 minutes; it decreased muscle tone in all 5 mice from 15 to 30 minutes, in 4 mice from 60 to 120 minutes and in 3 mice at 180 minutes.

Overall, 3-n-butylphthalide and ligustilide showed a dose dependent sedative and muscle relaxant effect.

### Example 4: Porsolt's swim test with 3-n-butylphthalide and ligustilide

The "Behavioural Despair Test" or "Persolt's Forced Swim Test" is a validated animal model for depression (see T. Nagatsu 2004 NeuroToxicology, 25:11-20, and Porsolt et al. 1977, Arch. Int. Pharmacodyn., 229: 327-336). It responds to enhancement of the transmission of several neurotransmitters including serotonine, dopamine and noradrenaline.

The test, which detects antidepressant activity, was carried out as described by Porsolt et al *supra.* Mice which are forced to swim in a situation from which they cannot escape rapidly become immobile. Antidepressants decrease the duration of immobility.

Mice were individually placed in a cylinder (Height = 24 cm, Diameter = 13 cm) containing 10 cm water (22°C) from which they could not escape. The mice were placed in the water for 6 minutes and the duration of immobility during the last 4 minutes was measured.

10 mice were studied per each of the four groups. The test was performed blind, i.e. the person carrying out the experiment was different from the person injecting the mice and didn't thus know to which of the four groups each mouse belonged.

3-n-butylphthalide (98.5% purity) was evaluated at 3 doses each: at 10, 30 and 80 mg/kg body weight, administered intraperitonally 30 minutes before the test, and compared with a control group. The thus administered 3-n-butylphthalide was dissolved in a saline solution containing 3 weight-% DMSO and 3 weight-% Tween® 80 (so called "vehicle"). To the control group the vehicle consisting of the saline solution containing 3 weight-% DMSO and 3 weight-% Tween® 80 was administered intraperitonally,

Results are shown in Table 4.

**Table 4: Reduction of "immobility time" with 3-n-butylphthalide.**

| **Group** | **Concentration of 3-n-butylphthalide** | **Duration of immobility [second] ± Standard Error of Mean** |
|---|---|---|
| | [**mg/kg body weight]** | |
| Group 1 | 0 (control group) | 157.4 ± 1.3.2 |
| Group 2 | 10 | 132.4 ± 9.3 |
| Group 3 | 30 | 128.0 ± 15.3 |
| Group 4 | 80 | 129.0 ± 15.4 |

3-n-butylphthalide between 10 and 80mg/kg showed a tendency to reduce the immobility time by approximately 18%.

**Table 5: Reduction of "immobility time" with ligustilide**

| **Group** | **Concentration of ligustilide** | **Duration of immobility [seconds] ± Standard Error of Mean** |
|---|---|---|
| | **[mg/kg body weight]** | |
| Group 1 | 0 (control group) | 158.7 ± 10.1 |
| Group 2 | 10 | 128.9 ± 15.1 |
| Group 3 | 30 | 164.2 ± 15.5 |
| Group 4 | 80 | 147.1.0 ± 12.1 |

Ligustilide at 10 mg/kg showed a tendency to reduce the immobility time by approx. 18%, but not at the higher doses.

### Example 5: Elevated Pluz Maze Test with 3-n-butylphthalide

The method, which detects anxiolytic activity, follows that described by Handley et al, 1984 (Naunyn-Schm Arch Pharm 387: 1-5. Rodents avoid open spaces (the open arms of an elevated plus-maze). Anxiolytics increase exploratory activity in the open amps.

The maze consists of 4 arms of equal length and width (14 x 5 cm) arranged in the form of a plus sign (+). Two opposite arms are enclosed by 12 cm high walls (closed arms). The 2 other arms have no walls (open arms). The maze is raised 56 cm above the floor. A mouse is placed in the centre of the plus-maze and left to explore for 5 minutes. The number of entries into the open and closed arms and the time spent in the open arms is recorded, 10 mice were studied per group. The test was performed blend.

3-n-Butylphthalide was tested at doses of 10, 30, and 80 mg/kg body weight.

The results are shown in Table 6.

**Table 6:**

| TREATMENT (mg/kg) p.o. -24 h. 5 h and 1h before test | OPEN ARM Number of | entries | OPEN ARM Time spent (s) | |
|---|---|---|---|---|
| | Mean ± sem | % change | Mean ± sem | % change |
| Vehicle | 2.4 ± 0.5 | - | 18.2 ± 4.9 | - |
| 3-n-Butylphthalide | (10) 3.5 ± 0.7 | +46% | 24.3 ± 4.6 | +34% |
| 3-n-Butylphthalide (30) | 3.1 ± 0.5 | +29% | 23.0 ± 3.8 | +26% |
| 3-n-Butylphthalide (80) | 3.0 ± 0.8 | +25% | 26.4 ± 5.3 | +45% |

3-n-butylphthalide showed a trend to moderately increase the number of entries and the time spent in the open arm. It had no effect on the entries into the closed arm. 3-n-butylphthalide thus has a moderate anxiolytic activity.

### Example 6: Marble Burying Test as test for anxiety like or obsessive compulsive behaviour

A celery seed oil extract rich in phthalides was tested in the marble burying test. A crude extract from celery seed oil (*Apium graveolens*) obtained by supercritical CO₂ fluid extraction of the seed was bought from Guanzhou Honsea Sunshine Bio Science & Technology, Guangzhou, 510620, Peopole's Republic of China. This extract (66.15g) was distilled for about 2 hours in a ca. 30 cm Kugelrohr at a temperature of 125°C, under a pressure of 0.02 mbar. The distillate contained 18.12% butylphthalide, 0.87% butylidenphthalide, 35.37% senkyunolide, 20.99% sedanolide, 2.23% C16-fatty acid and 3.14% oleic acid, based en the total weight of the celery seed oil extract.

"Defensive burying" behaviour was demonstrated by rats burying noxious objects, such as drinking spouts filled with a unpleasant-tasting liquid (Wilkie et al., 1979 J ExpAnal Behav. 31: 299-306) or shock prods (Pinel et al 1978, J Camp and Physiol Psych 92: 708-712.

Marble burying behaviour by mice is reported to be sensitive to a range of minor (e.g. diazepam) and major (e.g. haloperidol) tranquilisers (Broekkamp et al., 1985 Eur J Pharmacol. 126:223-229), in addition to SSRIs (e.g. fluvoxamine, fluoxetine, citalopram), tricyclic antidepressants (e.g. imipramine, desipramine) and selective noradrenaline uptake inhibitors (e.g. reboxetine), at doses which do not induce sedation. The model may reflect either anxiety-like-or obsessive-compulsive- behaviour (see De Boer et al, 2003 Eur J Pharmacol 463,145-161.

The marble burying test was performed in Type II cages (16 x 22 cm), which were filled with normal rodent bedding material, up to a depth of 4 cm, and the bedding was flattened gently by hand to ensure an even surface. Twelve marbles were placed, in a 3 x 4 configuration, evenly spaced, on the surface of the bedding.

At the start of the test, mice were individually placed into the test cages and left to explore for 30 min. At the end of the test, the mice were removed from the cages and the number of marbles which have been buried (i.e. at least 2/3 covered with bedding) were counted. Fresh cages and bedding are prepared prior to testing subsequent animals.

Celery seed oil extract was evaluated after sub-chronic oral administration at 50, 100, 200, 300 mg/kg with fluoxetine at 10mg/kg as reference substance and corn oil as vehicle. The celery seed oil extract fluoxetine and vehicle were administered by oral gavage 24, -5, -1 h before the test.

After each dose, mice were returned to their homecages until the next dose or beginning of the test.

### Results:

**Table 7: Effects of celery seed oil extract (Apium graveolens) and fluoxetine in the marble burying test in mice**

| TREATMENT | MARBLE BURYING TEST |
|---|---|
| (mg/kg) p.o., -24, -5, -1 h | % MARBLES BURIED |
| *Apium graveolens* (50) | -16 % |
| *Apium graveolens* (100) | -4 % |
| *Apium graveolens* (200) | -45 % |
| *Apium graveolens* (300) | -37 % |
| Fluoxetine (10) | - 78 % |

Celery seed oil extract showed a tendency to decrease the number of marbles covered as compared with the vehicle control. The effect was nearly half of that of the selective serotonin reuptake inhibitor fluoxetine.

### Example 7: Dopamine uptake inhibition by compounds of the formula I.

The actions of several neurotransmitters, including dopamine, are regulated through their rapid uptake and clearance from synaptic junctions by plasma membrane transport proteins. The dopamine transporter in central dopaminergic neurones is responsible for the recovery of up to 90% of released transmitter. The monoamine transporters are high affinity targets for a number of psychoactive agents such as cocaine, amphetamine, and antidepressants. These agents, by blocking transporters and consequently preventing neuronal uptake, elevate levels of extracellular neurotransmitter concentrations in both the central and peripheral nervous system, contributing to their behavioral and autonomic effects.

CHO-Ki/hDAT cells expressing the human dopamine transporter (hDAT) were plated before the assay. Cells (2 x 10⁵/ml) were incubated with the compound of the formula I (3-n-butylphthalide) and/or vehicle in modified Tris-HEPES buffer pH 7.1 at 25°C for 20 minutes before addition of 50 nM [³H]Dopamine for 10 minutes. Specific signal was determined in the presence of 10 µM nomifensine. Cells were then solubilized with 1% SDS lysis buffer. Reduction of [³H]Dopamine uptake by 50 per cent or more (≥ 50%) relative to vehicle controls indicates significant inhibitory activity. Compounds were screened at 10, 1, 0.1, 0.01 and 0.001 µM. These same concentrations were concurrently applied to a separate group of untreated cells and evaluated for possible compound-induced cytotoxicity only if significant inhibition of uptake was observed

| **Compound** | **IC₅₀** |
|---|---|
| *Nomifensine | 11 nM |
| 3-n-Butylphthalide | 22.0 µM |

| | |
|---|---|
| *Indicates standard reference agent used. | |

### References:

Giros, 1992 Mol. Pharmacol. 42:383-390, 1992.
Pristupa, et al 1994. Mol. Pharmacol. 45: 125-135.

### Example 8: Norepinephrine uptake inhibition by compounds of the formula I.

The actions of several neurotransmitters, including norepinephrine, are regulated through their rapid uptake and clearance from synaptic junctions by plasma membrane transport proteins. The norepinephrine transporter in central adrenergic neurones is responsible for the recovery of up to 90% of released transmitter. The monoamine transporters are high affinity targets for a number of psychoactive agents such as cocaine, amphetamine, and antidepressants. These agents, by blocking transporters and consequently preventing neuronal uptake, elevate levels of extracellular neurotransmitter concentrations in both the central and peripheral nervous system, contributing to their behavioral and autonomic effects.

Human recombinant norepinephrine transporter stably expressed dog kidney MDCK cells are plated one day before the assay. The cells (2 x 10⁵/ml) were preincubated with the compound of the formula I (3-n-butylphthalide) and/or vehicle in modified Tri-HEPES buffer pH 7.1 at 25°C for 20 minutes, then 25 nM [³H]Norepinephrine was added for 10 minutes incubation. Cells in the well were then rinsed twice, solubilized with 1% SDS lysis buffer and the lysate was counted to determine [³H]Norepinephrine uptake. Specific signal was determined in the presence of 10 µM desipramine. Reduction of [³H]Norepinephrine uptake by 50 percent or more (≥50%) relative to vehicle controls indicates significant inhibitory activity. Compounds were screened at 10, 1, 0.1, 0.01 and 0.001 µM. These same concentrations were concurrently applied to a separate group of untreated cells and evaluated for possible compound-induced cytotoxicity only if significant inhibition of uptake was observed.

### Reference Data:

| **Inhibitor** | **IC₅₀** |
|---|---|
| *Desipramine | 1.9 nM |
| 3-n-Butylphthalide | 23.7µM |

| | |
|---|---|
| *Indicates standard reference agent used. ^{◆} The experiment was carried out twice | |

### Reference:

Galliet al 1995. J. Exp. Biol. 198:2197-2212.

### Example 9: Preparation of a soft gelatin capsule

A soft gelatin capsule (500 mg) may be prepared comprising the following ingredients:

| **Ingredient** | **Amount per Capsule** |
|---|---|
| Celery seed oil extract | 500 mg |
| Lecithin | 50 mg |
| Soy bean oil | 250 mg |

Two capsules per day for 3 months may be administered to a human adult for the treatment of mild chronic dysthymia.

### Example 10: Preparation of a soft gelatin capsule

A soft gelatin capsule (600 mg) may be prepared comprising the following ingredients:

| **Ingredient** | **Amount per Capsule** |
|---|---|
| Celery seed oil extract | 200 mg |
| Evening primrose oil | 300 mg |
| Vitamin B₆ | 100 mg |

One capsule per day preferably at the second half of the menstrual cycle may be taken for 14 days for the treatment of premenstrual syndrome and premenstrual dysphoric disorder.

### Example 11: Preparation of a tablet

A 400 mg-tablet may be prepared comprising the following ingredients:

| **Ingredient** | **Amount per tablet** |
|---|---|
| 3-N-butylphthalide | 100 mg |
| Passion flower standardized extract | 150 mg |
| Green Tea Extract, e.g. TEAVIGO® from DSM Nutritional Products, Kaiseraugst, Switzerland | 150 mg |

For general well being, energizing and stress alleviation, one tablet may be taken twice daily for 3 months.

### Example 12: Preparation of an instant flavoured soft drink

| **Ingredient** | **Amount [g]** |
|---|---|
| 3-N-butylphthalide | 0.9 |
| Sucrose, fine powder | 922.7 |
| Ascorbic acid, fine powder | 2.0 |
| Citric acid anhydrous powder | 55.0 |
| Lemon flavour | 8.0 |
| Trisodium citrate anhydrous powder | 6.0 |
| Tricalciumphosphate | 5.0 |
| β-Carotene 1% CWS from DNP AG, Kaiseraugst, Switzerland | 0.4 |
| **Total amount** | **1000** |

All ingredients are blended and sieved through a 500µm sieve. The resulting powder is put in an appropriate container and mixed on a turbular blender for at least 20 minutes. For preparing the drink, 125 g of the obtained mixed powder are taken and filled up with water to one liter of beverage.

The ready-to-drink soft drink contains ca. 30 mg of 3-n-butylphthalide per serving (250 ml).

As a strengthener and for general well being 2 servings per day (240ml) may be drunk.

### Example 13: Preparation of a fortified non baked cereal bar

| **Ingredient** | **Amount [g]** |
|---|---|
| 3-N-butylphthalide | 0.95 |
| Sugar | 114.55 |
| Water | 54.0 |
| Salt | 1.5 |
| Glucose syrup | 130.0 |
| Invert sugar syrup | 95.0 |
| Sorbitol Syrup | 35.0 |
| Palm kernel fat | 60.0 |
| Baking fat | 40.0 |
| Lecithin | 1.5 |
| Hardenend palm-oil | 2.5 |
| Dried and cut apple | 63.0 |
| Cornflakes | 100.0 |
| Rice crispies | 120.0 |
| Wheat crispies | 90.0 |
| Roasted hazelnut | 40.0 |
| Skim milk powder | 45.0 |
| Apple flavour 74863-33 | 2.0 |
| Citric acid | 5.0 |
| **Total amount** | 1000 |

3-n-Butylphthalide is premixed with skim milk powder and placed in a planetary bowl mixer. Cornflakes and rice crispies are added and the total is mixed gently. Then the dried and cut apples are added. In a first cooking pot sugar, water and salt are mixed in the amounts given above (solution 1). In a second cooking pot glucose, invert and sorbitol syrup are mixed in the amounts given above (solution 2). A mixture of baking fat, palm-kernel fat, lecithin and emulsifier is the fat phase. Solution 1 is heated to 110°C. Solution 2 is heated to 113°C and then cooled in a cold water bath. Afterwards solution 1 and 2 are combined. The fat phase is melted at 75°C in a water bath. The fat phase is added to the combined mixture of solution 1 and 2. Apple flavour and citric acid are added to the liquid sugar-fat mix. The liquid mass is added to the dry ingredients and mixed well in the planetary bowl mixer. The mass is put on a marble plate and rolled to the desired thickness. The mass is cooled down to room temperature and cut into pieces. The non baked cereal bar contains ca. 25 mg oregano extract per serving (30 g). For general well-being and energizing 1-2 cereal bars may be eaten per day.

### Example 14: Dry dog feed comprising 3-n-butvlphthalide or ligustilide for relieving stress and revitalizing the dog

Commercial basal diet for dogs (e.g. Mera Dog "Brocken", MERA-Tiernahrung GmbH, Marienstraße 80-84, D-47625 Kevelaer-Wetten, Germany) is sprayed with a solution of 3-n-butylphthalide or ligustilide in an amount sufficient to administer to a subject a daily dose of 50 mg of 3-n-butylphthalide or ligustilide per kg body weight. The food composition is dried to contain dry matter of about 90 % by weight. For an average dog of 10 kg body weight to consume approx. 200g dry feed per day, the dog food contains approx. 2500 mag 3-n-butylphthalide or ligustilide per kg food. For heavier dogs, the feed mix is prepared accordingly.

### Example 15: Wet cat food comprising 3-n-butylphthalide or ligustilide

Commercial basal diet for cats (e.g. Happy Cat "Adult", Tierfeinnahrung, Südliche Hauptstraße 38, D-86517 Wehringen, Germany) is mixed with a solution of 3-n-butylphthalide or ligustilide in an amount sufficient to administer to a subject a daily dose of 100 mg of 3-n-butylphthalide or ligustilide per kg body weight. For an average cat of 5 kg of body weight to consume approx. 400 g of wet food per day, the cat food contains 1250 mg/kg.

## Claims

1. A dietary or pharmaceutical composition comprising ligustilide for use in a method of treatment or prophylaxis of depression, generalized anxiety disorders, dysphoria, obsessive-compulsive behaviour, and mood disorder.

2. A composition for the use as in Claim 1 wherein the composition is in the form of a food in solid or liquid form such as soups or dairy products, in the form of fortified food such as cereal bars and bakery items such as cakes and cookies, in the form of dietary supplements such as tablets, pills, granules, dragées, capsules, and effervescent formulations, or in the form of non-alcoholic drinks such as soft drinks, sport drinks, fruit juices, lemonades, teas and milk based drinks.

3. A composition for the use as in Claim 1 or 2 which is used in animals.

4. Non-therapeutic use of dietary composition comprising ligustilide as
a mood/vitality improver, stress reliever, reducer of anxiety, a reducer of tension, sadness, unhappiness, discontentedness, or irritability;
or for normalizing and maintaining circadian rhythms, or alleviating or preventing symptoms associated with disturbed circadian rhythms, wherein the circadian rhythms are disturbed by jet-lags or shift working.

5. Use according to Claim 4 wherein the composition is in the form of a food in solid or liquid form such as soups or dairy products, in the form of fortified food such as cereal bars and bakery items such as cakes and cookies, in the form of dietary supplements such as tablets, pills, granules, dragées, capsules, and effervescent formulations, or in the form of non-alcoholic drinks such as soft drinks, sport drinks, fruit juices, lemonades, teas and milk based drinks.

6. Use according to Claim 4 or 5 which is used in animals.

## Patentansprüche

1. Ligustilid enthaltende Nahrungszusammensetzung bzw. pharmazeutische Zusammensetzung zur Verwendung bei einem Verfahren zur Behandlung oder Prophylaxe von Depression, allgemeinen Angststörungen, Dysphorie, Zwangsneurose und affektiver Psychose.

2. Zusammensetzung für die Verwendung nach Anspruch 1, wobei die Zusammensetzung in Form eines Nahrungsmittels in fester oder flüssiger Form wie Suppen oder Milchprodukten, in Form von angereicherten Nahrungsmitteln wie Cerealienriegeln und Backprodukten wie Kuchen und Keksen, in Form von Nahrungsergänzungsmitteln wie Tabletten, Pillen, Granulaten, Dragees, Kapseln und Brauseformulierungen oder in Form nichtalkoholischer Getränke, wie Softdrinks, Sportgetränke, Fruchtsäfte, Limonaden, Tees und Getränken auf Milchbasis vorliegt.

3. Zusammensetzung zur Verwendung nach Anspruch 1 oder 2, welche bei Tieren zur Anwendung kommt.

4. Nichttherapeutische Verwendung einer Ligustilid enthaltenden Nahrungsmittelzusammensetzung als Mittel zum Verbessern der Stimmung/Vitalität, Mittel zum Stressabbau, Mittel zum Angstabbau, Mittel zum Abbau von Spannung, Traurigkeit, Unglücklichsein, Unzufriedenheit bzw. Reizbarkeit;
oder zur Normalisierung und Aufrechterhaltung des Tag-Nacht-Rhythmus oder zur Linderung bzw. Prävention von mit einem gestörten Tag-Nacht-Rhythmus assoziierten Symptomen, wobei die Tag-Nacht-Rhythmen durch Jetlag oder Schichtarbeit gestört sind.

5. Verwendung nach Anspruch 4, wobei die Zusammensetzung in Form eines Nahrungsmittels in fester oder flüssiger Form wie Suppen oder Milchprodukten, in Form von angereicherten Nahrungsmitteln wie Cerealienriegeln und Backprodukten wie Kuchen und Keksen, in Form von Nahrungsergänzungsmitteln wie Tabletten, Pillen, Granulaten, Dragees, Kapseln und Brauseformulierungen oder in Form nichtalkoholischer Getränke, wie Softdrinks, Sportgetränke, Fruchtsäfte, Limonaden, Tees und Getränken auf Milchbasis vorliegt.

6. Verwendung nach Anspruch 4 oder 5, welche bei Tieren zur Anwendung kommt.

## Revendications

1. Composition pharmaceutique ou diététique comprenant du ligustilide, pour une utilisation dans une méthode de traitement ou de prophylaxie de la dépression, des troubles d'anxiété généralisée, de la dysphorie, du trouble obsessionnel-compulsif, et des troubles de l'humeur.

2. Composition pour une utilisation selon la revendication 1, dans laquelle la composition se trouve sous la forme d'un aliment sous forme solide ou liquide tel que les soupes ou les produits laitiers, sous la forme d'aliments fortifiés tels que les barres de céréales et les articles de panification tels que les gâteaux et les cookies, sous la forme de compléments alimentaires tels que les comprimés, les pilules, les granulés, les dragées, les capsules et les formulations effervescentes, ou sous la forme de boissons non alcoolisées telles que les boissons gazeuses, les boissons pour sportifs, les jus de fruits, les limonades, les thés et des boissons à base de lait.

3. Composition pour une utilisation selon la revendication 1 ou 2, qui est utilisée chez l'animal.

4. Utilisation non thérapeutique d'une composition diététique comprenant du ligustilide comme
améliorant de l'humeur/vitalité, soulagement de stress, réducteur d'anxiété, réducteur de tension, tristesse, chagrin, mécontentement ou irritabilité ;
ou pour normaliser et maintenir les rythmes circadiens, ou soulager ou empêcher les symptômes associés aux rythmes circadiens perturbés, où les rythmes circadiens sont perturbés par les fatigues dues au décalage horaire ou le travail en équipe.

5. Utilisation selon la revendication 4, dans laquelle la composition se trouve sous la forme d'un aliment sous forme solide ou liquide tel que les soupes ou les produits laitiers, sous la forme d'aliments fortifiés tels que les barres de céréales et les articles de panification tels que les gâteaux et les cookies, sous la forme de compléments alimentaires tels que les comprimés, les pilules, les granulés, 1 les dragées, les capsules et les formulations effervescentes, ou sous la forme de boissons non alcoolisées telles que les boissons gazeuses, les boissons pour sportifs, les jus de fruits, les limonades, les thés et des boissons à base de lait.

6. Utilisation selon la revendication 4 ou 5, qui est utilisée chez l'animal.
